# EUROPÄISCHE PATENTANMELDUNG

(11) **EP 3 184 531 A1**
(43) Veröffentlichungstag der Anmeldung: **28.06.2017**
(21) Anmeldenummer: 15201638.2
(22) Anmeldetag: 21.12.2015
(51) Int. Cl.: C07F 9/6509, C07F 9/6571, C07F 15/00

(54) **BIDENTATE DIPHOSPHORAMIDITE MIT EINER PIPERAZINGRUPPE ALS LIGANDEN FÜR DIE HYDROFORMYLIERUNG**

(71) Anmelder: Evonik Degussa GmbH, 45128 Essen (DE)
(72) Erfinder: DYBALLA, Katrin Marie, 45657 Recklinghausen (DE); FRANKE, Robert, 45772 Marl (DE); FRIDAG, Dirk, 45721 Haltern am See (DE); HESS, Dieter, 45770 Marl (DE); GEILEN, Frank, 45721 Haltern am See (DE); MORALES TORRES, Galina, 18057 Rostock (DE); SELENT, Detlef, 18059 Rostock (DE); BÖRNER, Armin, 18059 Rostock (DE)

(57) **Zusammenfassung**

Die Erfindung betrifft Rh-, Ru-, Co- und Ir-Komplexe umfassend bidentate Diphosphoramidite als Liganden und deren Verwendung Katalysatoren für die Hydroformylierung von Olefinen. Die Erfindung betrifft außerdem ein Verfahren zur Herstellung eines Aldehyds aus einem Olefin unter Verwendung der genannten Komplexe bzw. Liganden.

## Beschreibung

Die Erfindung betrifft Rh-, Ru-, Co- und Ir-Komplexe umfassend bidentate Diphosphoramidite als Liganden und deren Verwendung als Katalysatoren für die Hydroformylierung von Olefinen. Die Erfindung betrifft außerdem ein Verfahren zur Herstellung eines Aldehyds aus einem Olefin unter Verwendung der genannten Komplexe bzw. Liganden.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Die Hydroformylierung ist eine der mengenmäßig bedeutendsten homogenen Katalysen im industriellen Maßstab. Die dabei erzeugten Aldehyde sind wichtige Zwischen- bzw. Endprodukte in der chemischen Industrie (B. Cornelis, W.A. Herrmann, "Applied Homogeneous catalysis with organometallic compounds", Vol. 1 & 2, VCH, Weinheim, New York, 1996; R. Franke, D. Selent, A. Börner, Chem. Rev. 2012, 112, 5675).

Als Katalysatoren in der Hydroformylierung werden häufig Verbindungen der Übergangsmetalle der VIII. Gruppe des Periodensystems der Elemente verwendet. Von besonderer Bedeutung sind dabei Rh-Katalysatoren. Die Katalysatoren umfassen ferner geeignete Liganden, beispielsweise Verbindungen aus den Klassen der Phosphine, Phosphite und Phosphonite mit jeweils dreiwertigem Phosphor. Diese dreiwertigen Phosphorverbindungen werden meist zur Steuerung von Aktivität und Regioselektivität des Katalysators eingesetzt. Da die Hydroformylierung, außer im Fall von Ethylen als Edukt, zu einer Mischung isomerer Produkte führt, nämlich n-Aldehyden (linearen Aldehyden) und iso-Aldehyden (verzweigten Aldehyden), ist neben der Reaktionsgeschwindigkeit und damit Ausbeuten, insbesondere die Selektivität bei der Bildung von n- bzw. iso-Produkten ein entscheidender Parameter der Hydroformylierungsreaktion.

Phosphoramidite, also Verbindungen, welche anstellte einer P-O- über eine oder mehrere P-N-Bindung verfügen, wurden bisher nur selten als Liganden bei der Hydroformylierung eingesetzt.

Phosphoramidite, welche eine Phenyl-Phenyl-Einheit aufweisen, wurden in der Literatur beschrieben (J. Mazuela et al., Tetrahedron Asymmetry, 2010, 21(17):2153 - 2157, Seite 2154, Verbindung L2). Van Leuwwen und Mitarbeiter untersuchten erstmals monodentate Phosphoramidite in der Hydroformylierung (A. van Rooy, D. Burgers, P. C. J. Kamer, P. W. N. M. van Leeuwen, Recl. Trav. Chim. Pays-Bas 1996, 115, 492). Insgesamt wurden bei hohen Ligand/Rhodium-Verhältnissen von bis zu 1000:1 nur mäßige katalytische Eigenschaften beobachtet.

WO 2007/031065 A1 offenbart die Verwendung von chiralen Phosphoramiditen für asymmetrische Katalysen, ohne allerdings Ausführungsbeispiele für die Hydroformylierung anzugeben.

Chirale bidentate Liganden mit jeweils einer Phosphoramiditeinheit wurden verschiedentlich in der asymmetrischen Hydroformylierung verwendet (J. Mazuela, O. Pämies, M. Dieguez, L. Palais, S. Rosset, A. Alexakis, Tetrahedron: Asymmetry 2010, 21, 2153-2157; Y. Yan, X. Zhang, J. Am. Chem. Soc. 2006, 128, 7198-7202; Z. Hua, V. C. Vassar, H. Choi, I. Ojima, PNAS 2004, 13, 5411-5416).

Der vorliegenden Erfindung liegt die Aufgabe zugrunde, neue Liganden und Katalysatoren für die Hydroformylierung bereitzustellen, welche eine hohe Ausbeute gewährleisten und es erlauben, dass n/iso-Verhältnis zu steuern.

Diese Aufgabe wird gelöst durch einen Komplex umfassend Rh, Ru, Co oder Ir und eine Verbindung nach einer der allgemeinen Formeln (**I**) oder (**II**) 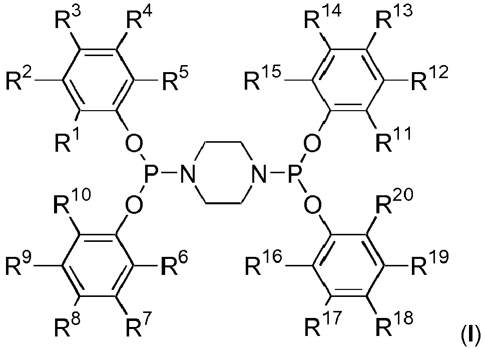 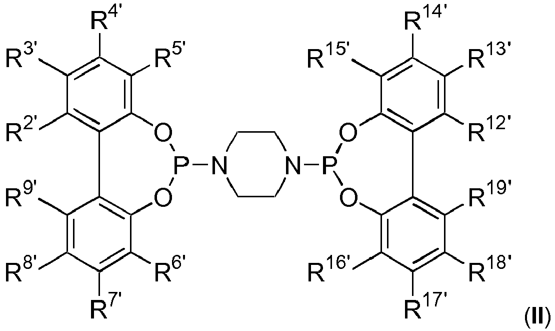 wobei R¹, R², R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰ unabhängig voneinander ausgewählt sind aus -H, -(C₁-C₁₂)-Alkyl, -O-(C₁-C₁₂)-Alkyl, -(C₆-C₂₀)-Aryl, -O-(C₆-C₂₀)-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -N[(C₁-C₁₂)-Alkyl]₂;
und R^{2'}, R^{3'}, R^{4'}, R^{5'}, R^{6'}, R^{7'}, R^{8'}, R^{9'}, R^{12'}, R^{13'}, R^{14'}, R^{15'}, R^{16'}, R^{17'}, R^{18'}, R^{19'} unabhängig voneinander ausgewählt sind aus -H, -(C₁-C₁₂)-Alkyl, -O-(C₁-C₁₂)-Alkyl, -(C₆-C₂₀)-Aryl, -O-(C₆-C₂₀)-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -N[(C₁-C₁₂)-Alkyl]₂.

Die erfindungsgemäßen Komplexe eignen sich als Katalysatoren für die Hydroformylierung von Olefinen, beispielsweise von Octenen oder Penten, mit denen sich hohe Ausbeuten erzielen lassen. Des Weiteren kann durch geeignete Auswahl der Katalysatoren das n/iso-Verhältnis gezielt gesteuert werden.

Die erfindungsgemäßen Katalysatoren zeichnen sich durch die Verbindung gemäß den Formeln (**I**) und (**II**) aus, welche bislang nicht bei der Hydroformylierung von Olefinen zum Einsatz kamen.

Rhodium-Komplexe sind besonders geeignete Katalysatoren für die Hydroformylierung. Vorzugsweise umfassen die erfindungsgemäßen Katalysatoren deshalb Rh.

R¹, R², R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰ sind vorzugsweise unabhängig voneinander ausgewählt aus -H, -(C₁-C₁₂)-Alkyl, -O-(C₁-C₁₂)-Alkyl, -(C₆-C₂₀)-Aryl, -O-(C₆-C₂₀)-Aryl, - Halogen.

In einer bevorzugten Ausführungsform sind R¹, R², R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰ unabhängig voneinander ausgewählt aus -H, -(C₁-C₁₂)-Alkyl, -O-(C₁-C₁₂)-Alkyl, -(C₆-C₂₀)-Aryl, -O-(C₆-C₂₀)-Aryl.

In einer besonders bevorzugten Ausführungsform sind R¹, R², R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰ unabhängig voneinander ausgewählt aus -H,-(C₁-C₁₂)-Alkyl, -O-(C₁-C₁₂)-Alkyl.

In einer weiteren besonders bevorzugten Ausführungsform sind R¹, R², R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰ unabhängig voneinander ausgewählt aus -H, -(C₁-C₆)-Alkyl.

R^{2'}, R^{3'}, R^{4'}, R^{5'}, R^{6'}, R^{7'}, R^{8'}, R^{9'}, R^{12'}, R^{13'}, R^{14'}, R^{15'}, R^{16'}, R^{17'}, R^{18'}, R^{19'} sind vorzugsweise unabhängig voneinander ausgewählt aus -H, -(C₁-C₁₂)-Alkyl, -O-(C₁-C₁₂)-Alkyl, -(C₆-C₂₀)-Aryl, -O-(C₆-C₂₀)-Aryl, - Halogen.

In einer bevorzugten Ausführungsform sind R^{2'}, R^{3'}, R^{4'}, R^{5'}, R^{6'}, R^{7'}, R^{8'}, R^{9'}, R^{12'}, R^{13'}, R^{14'}, R^{15'} R^{16'}, R^{17'}, R^{18'}, R^{19'} unabhängig voneinander ausgewählt aus -H, -(C₁-C₁₂)-Alkyl, -O-(C₁-C₁₂)-Alkyl, -(C₆-C₂₀)-Aryl, -O-(C₆-C₂₀)-Aryl.

In einer besonders bevorzugten Ausführungsform sind R^{2'}, R^{3'}, R^{4'}, R^{5'}, R^{6'}, R^{7'}, R^{8'}, R^{9'}, R^{12'}, R^{13'}, R^{14'}, R^{15'}, R^{16'}, R^{17'}, R^{18'}, R^{19'} unabhängig voneinander ausgewählt aus -H, -(C₁-C₁₂)-Alkyl, -O-(C₁-C₁₂)-Alkyl.

In einer weiteren besonders bevorzugten Ausführungsform sind R^{2'}, R^{3'}, R^{4'}, R^{5'}, R^{6'}, R^{7'}, R^{8'}, R^{9'}, R^{12'}, R^{13'}, R^{14'}, R^{15'}, R^{16'}, R^{17'}, R^{18'}, R^{19'} unabhängig voneinander ausgewählt aus -H, -(C₁-C₆)-Alkyl.

In einer weiteren besonders bevorzugten Ausführungsform stehen R¹, R², R⁴, R⁷, R⁹, R¹⁰, R¹¹, R¹², R¹⁴, R¹⁷, R¹⁹, R²⁰ jeweils für H.

In einer weiteren besonders bevorzugten Ausführungsform stehen R^{2'}, R^{4'}, R^{7'}, R^{9'}, R^{12'}, R^{14'}, R^{17'}, R^{19'} jeweils für H.

In einer weiteren besonders bevorzugten Ausführungsform sind die Benzol- bzw. Dibenzolringe der Liganden (**I**) und (**II**) jeweils in ortho- und/oder para-Position subsituiert.

In einer Ausführungsform weist die Verbindung die allgemeine Formeln (**I**) auf.

In einer weiteren Ausführungsform weist die Verbindung die allgemeine Formeln (**II**) auf.

Die erfindungsgemäßen Katalysatoren umfassen somit vorzugsweise Rh, Ru, Co oder Ir, insbesondere aber Rh, und eine Verbindung gemäß einer der Formeln (**III**) oder (**IV**): 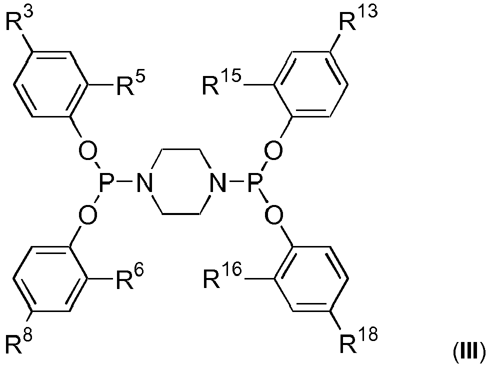 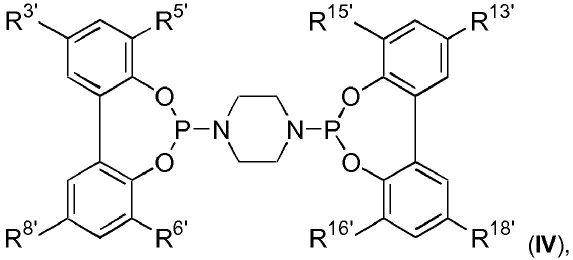
wobei R³, R⁵, R⁶, R⁸, R¹³, R¹⁵, R¹⁶, R¹⁸ unabhängig voneinander ausgewählt sind aus -H,-(C₁-C₁₂)-Alkyl, -O-(C₁-C₁₂)-Alkyl, -(C₆-C₂₀)-Aryl, -O-(C₆-C₂₀)-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -N[(C₁-C₁₂)-Alkyl]₂;
und R^{3'}, R^{5'}, R^{6'}, R^{8'}, R^{13'}, R^{15'}, R^{16'}, R^{18'} unabhängig voneinander ausgewählt sind aus -H,-(C₁-C₁₂)-Alkyl, -O-(C₁-C₁₂)-Alkyl, -(C₆-C₂₀)-Aryl, -O-(C₆-C₂₀)-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -N[(C₁-C₁₂)-Alkyl]₂.

Besonders bevorzugt sind R³, R⁵, R⁶, R⁸, R¹³, R¹⁵, R¹⁶, R¹⁸ in diesem Fall unabhängig voneinander ausgewählt aus -H, -(C₁-C₁₂)-Alkyl, -O-(C₁-C₁₂)-Alkyl.

Besonders bevorzugt sind R^{3'}, R^{5'}, R^{6'}, R^{8'}, R^{13'}, R^{15'}, R^{16'}, R^{18'} in diesem Fall unabhängig voneinander ausgewählt aus -H, -(C₁-C₁₂)-Alkyl, -O-(C₁-C₁₂)-Alkyl.

In einer Ausführungsform weist die Verbindung die allgemeine Formeln (**III**) auf.

In einer weiteren Ausführungsform weist die Verbindung die allgemeine Formeln (**IV**) auf.

Es hat sich gezeigt, dass die folgenden Verbindungen (**4**) bis (**7**) besonders geeignete Liganden der Formeln (**I**) und (**II**) darstellen. Die erfindungsgemäßen Komplexe umfassen deshalb vorzugsweise eine der Verbindungen (**4**), (**5**), (**6**) und (**7**):

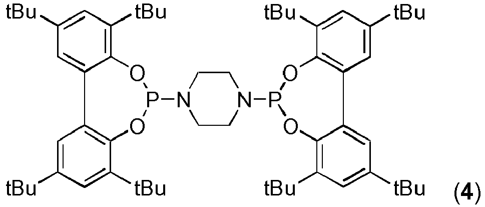

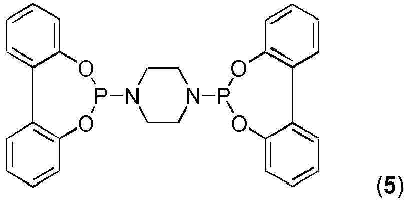

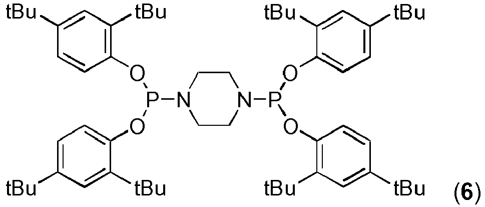

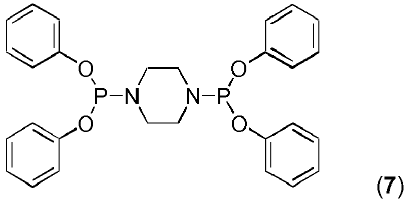

In einer Ausführungsform handelt es sich bei den Komplexen um einen Komplex gemäß der Formel **8** oder **9**:

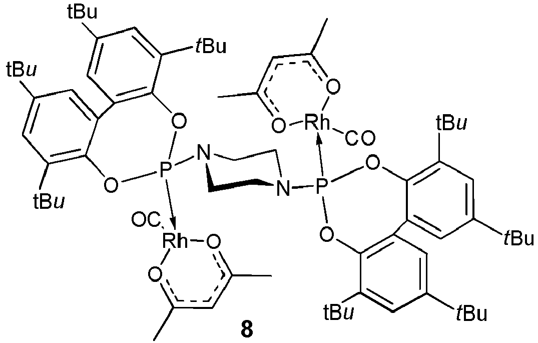

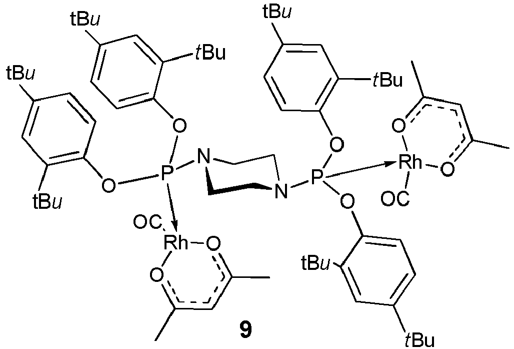

Die Erfindung betrifft außerdem die Verwendung des erfindungsgemäßen Komplexes bzw. der Verbindungen gemäß einer der Formeln (**I**) und (**II**) zur Katalyse einer Hydroformylierungsreaktion. Insbesondere betrifft dies die Hydroformylierung von Olefinen mit 2 bis 20 Kohlenstoffatomen, bevorzugt 2 bis 12 Kohlenstoffatomen, besonders bevorzugt 4 bis 10 Kohlenstoffatomen.

Die Erfindung betrifft außerdem ein Verfahren zur Herstellung eines Aldehyds, umfassend die Verfahrensschritte:
a) Vorlegen eines Olefins,
b) Zugabe eines erfindungsgemäßen Komplexes oder
   einer Verbindung nach einer der Formeln (I) und (II) und einer Katalysatorvorstufe umfassend einen Rh-, Ru-, Co- oder Ir-Komplex,
c) Zuführen von Wasserstoff und Kohlenstoffmonoxid,
d) Erwärmen des Reaktionsgemisches, wobei das Olefin zu einem Aldehyd umgesetzt wird. Hierbei können die Verfahrensschritte a) bis d) in beliebiger Reihenfolge erfolgen.

Die Edukte des erfindungsgemäßen Verfahrens sind Olefine oder Gemische von Olefinen, insbesondere von Olefinen mit 2 bis 20 Kohlenstoffatomen, bevorzugt 2 bis 12 Kohlenstoffatomen, besonders bevorzugt 4 bis 10 Kohlenstoffatomen.
Besonders geeignet sind Monoolefine mit einer endständigen Kohlenstoff-Kohlenstoff-Doppelbindung.

Geeignete Olefine sind beispielsweise 1-Propen, 1- oder 2-Penten, 2-Methyl-1-buten, 2-Methyl-2-buten, 3-Methyl-1-buten, 1-, 2- oder 3-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabutan) sowie durch Cooligomerisierung von Olefinen mit unterschiedlicher Anzahl von Kohlenstoffatomen (bevorzugt 2 bis 4) hergestellte Olefingemische.

In einer bevorzugten Variante des Verfahrens wird als Katalysator ein erfindungsgemäßer Komplex umfassend Rh eingesetzt.

Der Katalysatorkomplex kann auch *in situ* gebildet werden, indem in Schritt b) eine Verbindung nach einer der Formeln (**I**) oder (**II**), die als Ligand fungiert, und eine Katalysatorvorstufe umfassend einen Rh-, Ru-, Co- oder Ir-Komplex zugegeben werden. Durch eine Ligandenaustauschreaktion zwischen den Liganden der Katalysatorvorstufe und der Verbindung nach einer der Formeln (**I**) und (**II**) wird hierbei der erfindungsgemäße Katalysatorkomplex gebildet.

Hierbei kann auch ein Überschuss an Liganden verwendet werden, so dass nach Bildung des katalytischen Komplexes nicht zwangsläufig jeder Ligand in Form eines Ligand-MetallKomplexes vorliegt, sondern ein Teil der zugegebenen Liganden ungebunden im Reaktionsgemisch enthalten sind.

Das molare Verhältnis von der Verbindung nach einer der Formeln (**I**) oder (**II**) zum Metallatom der Katalysatorvorstufe liegt vorzugsweise im Bereich von 40:1 zu 1:1, bevorzugt 20:1 zu 1:1, besonders bevorzugt 5:1 zu 1:1.

Die Katalysatorvorstufe ist vorzugsweise ein Rh-, Ru-, Co- oder Ir-Komplex umfassend einen Liganden ausgewählt aus Acetylacetonat (acac), Acetat (OAc) und Chlorid. Besonders bevorzugt handelt es sich bei der Katalysatorvorstufe um einen Rh-Komplex.

Vorzugsweise handelt es sich bei der Katalysatorvorstufe um Rhodiumcarbonyle, Rhodiumnitrat, Rhodiumchlorid, Rh(CO)₂(acac) (acac = Acetylacetonat), Rhodiumacetat oder Rhodiumcarboxylate, beispielsweise Rhodiumoctanoat, beonders bevorzugt Rh(acac)(CO)₂.

Die Umsetzung des Olefins zum Aldehyd geschieht vorzugsweise bei einer Temperatur von 80 °C bis 200 °C, bevorzugt 90 °C bis 180 °C, besonders bevorzugt 100 °C bis 160 °C.

Die Umsetzung des Olefins zum Aldehyd geschieht vorzugsweise bei einem Druck von 1 bar bis 300 bar, bevorzugt 15 bar bis 250 bar, besonders bevorzugt 15 bar bis 50 bar.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl-, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Halogen als Substituent an Alkyl oder Aryl umfasst Fluor, Chlor, Brom und Jod, wobei Chlor und Fluor besonders bevorzugt sind.

Alle Erläuterungen zum Ausdruck -(C₁-C₁₂)-Alkyl in den vorgenannten erfindungsgemäßen Strukturen der Selena-Phosphite und Selenodiarylen gelten auch für die Alkylgruppen in -O-(C₁-C₁₂)-Alkyl, also in -(C₁-C₁₂)-Alkoxy.

Bevorzugt sind unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen in den vorgenannten Strukturen der Selena-Phosphite und Selenodiarylen können in Abhängigkeit von ihrer Kettenlänge, einen oder mehrere Substituenten aufweisen. Die Substituenten sind vorzugsweise unabhängig voneinander ausgewählt aus
-(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl. Diese Definition gilt für alle substituierten Alkyl- oder Alkyoxygruppen der vorliegenden Erfindung.

Bevorzugt sind unsubstituierte -O-(C₆-C₂₀)-Gruppen.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Nachfolgend wird die Erfindung näher an Beispielen erläutert, ohne die Erfindung auf die Ausführungsbeispiele zu beschränken.

### Beispiele

Die folgenden Beispiele illustrieren die Erfindung.

### Allgemeine Methoden

Alle Reaktionen wurden unter einer inerten Atmosphäre (Argon 5.0) unter Verwendung von Standard-Schlenk-Technik durchgeführt. Die Lösungsmittel wurden durch konventionelle Verfahren getrocknet und unter Argon destilliert. Wenn möglich, wurden die Reaktionen durch NMR-Spektroskopie überwacht. Die unten aufgeführten Ausbeuten sind isolierte Ausbeuten, Schmelzpunkte sind nicht korrigiert.

NMR-Spektren wurden bei Raumtemperatur mit einem Bruker AV 300, bzw. AV 400 MHz Spektrometer aufgenommen. Die chemischen Verschiebungen sind in ppm relativ zu TMS, Lösungsmittelsignale (Dichlormethan, δ_{H} = 5,32 ppm, δ_{C} = 53,8 ppm; Tetrahydrofuran δ_{H} = 3,58; 1,73 ppm; δ_{C} = 67,5; 25,37 ppm) wurden als sekundäre Referenz für ¹H- und ¹³C-NMR-Spektroskopie verwendet. Für die ³¹P-NMR-Spektren wurde externe H₃PO₄ als Standard verwendet.

Die IR-Spektren wurden an einem Nicolet 380 FT-IR angefertigt. Hochauflösende Massenspektrometrie (HRMS) wurde auf einem Agilent 6210 E1969A TOF-Spektrometer aufgenommen. Nur die Messungen mit einer mittleren Abweichung von der theoretischen Masse von ± 2 mDa wurden als korrekt berücksichtigt.

Röntgenbeugungsdaten von Einkristallen der Verbindungen **6** wurden auf einem Bruker APEX II Kappa Duo Diffraktometer aufgenommen. Die Strukturen wurden mit direkten Methoden mit Hilfe der SHELXS-97-Programm (G.M. Sheldrick, Acta. Crystallogr. Sect. A. 64 (2008) 112-122) und mit voller Matrix der kleinsten Quadrate Verfahren gegen F² verfeinert mit Hilfe der SHELXL-2014-Programm (G.M. Sheldrick, Acta. Crystallogr. Sect. C. 71 (2015) 3-8) gelöst.

Die Gaschromatographie wurde auf einem HP 5890 Series II unter Verwendung einer PONA Säule (0,5 mm; Länge 50 m; 0,2 mm Durchmesser) durchgeführt. Alle Reaktionen wurden durch Dünnschichtchromatographie (Kieselgel 60, F₂₅₄, E. Merck KGAG) verfolgt. Als Lösungsmittel-Systeme (v / v) wurden verwendet: Hexan-CH₂Cl₂ 1: 1 (*A₁*), bzw. 5: 1 (*A₂*); Hexan-EtOAc 99: 1 (*B₁*). Die Detektion erfolgte durch UV-Fluoreszenz (λ = 254 nm, λ = 365 nm).

Präparative Flash-Chromatographie wurde durch Verwendung von gepackten Säulen (Kieselgel, RediSep) mit einem CombiFlash-Rf System (Teledyne ISCO) durchgeführt.

### Synthese der Phosphorchloridite 1 - 3

Die Phosphorchloridite **1** (E. Benetskiy, S. Lühr, M. Vilches-Herrera, D. Selent, H. Jiao, L. Domke, K. Dyballa, R. Franke, A. Börner, ACS Catal. 4 (2014) 2130-2136 ), **2** [V.N. Tsarev, A.A. Kabro, S.K. Moiseev, V.N. Kalinin, O.G. Bondarev, V.A. Davankov, K.N. Gavrilov, Russ.Chem.Bull., Int.Ed. 53 (2004) 814-818; D. J. Frank, A. Franzke, A. Pfaltz, Chem. Eur. J. 19 (2013) 2405 - 2415; O. Lot, I. Suisse 1, A. Mortreux, F. Agbossou, J. Mol. Catal. A: Chem. 164 (2000) 125-130] und **3** (L. P. J. Burton, US 4739000 A (1988)) wurden entsprechen der Literatur hergestellt.

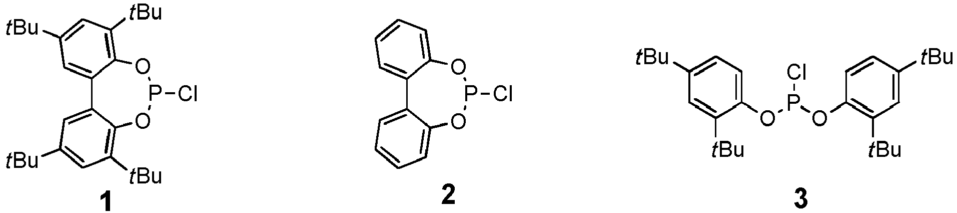

### Synthese der Phosphoramidite 4, 5 und 6.

Allgemeine Arbeitsvorschrift vgl. B. L. Feringa, J. F. Teichert, Angew. Chem. Int. Ed. 49 (2010) 2486-2528; E. Balaraman, K. C. Kumara Swamy, Tetrahedron: Asymmetry 18 (2007) 2037-2048; M. Rodriguez i Zubiri, A.M.Z. Slawin, M. Wainwright, J. Derek Woollins, Polyhedron. 21 (2002) 1729-1736.

Eine Lösung der entsprechenden Phosphorchloridite (2,0 mmol) in THF (20 ml) wurde tropfenweise über einen Zeitraum von 30 min zu einer eisgekühlten Lösung des Amins (1,0 mmol) und Triethylamin (4,0 mmol) in THF (30 ml) gegeben. Nach 15 min ließ man die Lösung langsam auf Raumtemperatur erwärmen und setzte das Rühren über Nacht fort, während dieser Zeit fiel Triethylammoniumhydrochlorid aus der farblosen Lösung aus und konnte durch Filtration abgetrennt werden. Nach Entfernen des Lösungsmittels im Vakuum wurde das Rohprodukt durch Flash-Chromatrography gereinigt, und die Amidite konnten als weiße Feststoffe isoliert werden. Alle Liganden sind leicht verfügbar und können im Gramm-Maßstab hergestellt werden.

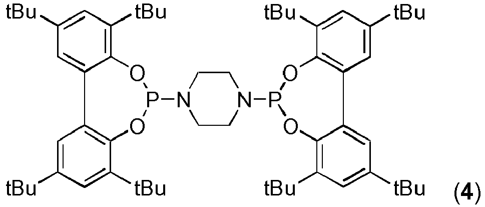

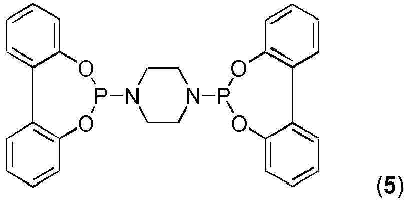

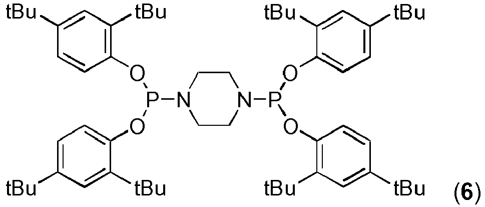

### 1,4-Bis(2,4,8,10-tetra-tert-butyldibenzo[d,f][1,3,2]dioxaphosphepin-6-yl)piperazin (4)

Vgl. a) M. Rasberger, EP 5500 A1. (1979); b) M. Rasberger, US 4301061 A. (1981); c) T. Shinya, P. Yukihiro, S. Motohiko, F. Kanako, S. Manji, Y. Tetsuo, Jpn. Kokai Tokkyo Koho , JP 07070158 A. (1995).

Ausbeute: 67%; weiße Kristalle; Schmp. 341-343 °C (Zersetzung); R*_{f}*-Wert 0,42 (System *B₁*); ³¹P-NMR (CD₂Cl₂): δ 143,48; ¹H-NMR (CD₂Cl₂; 300,13 MHz): δ 7,41; 7,13 (2br.s, 8H, ArH), 3,10-2,75 (m, 8H, NC*H*₂); 1,47; 1,34 (2s, 72H, C(C*H*₃)₃); ¹³C-NMR (CD₂Cl₂; 75,46 MHz): δ 147,48 (d, ²*J*_{C,P} = 5,68 Hz; ArC-OP); 146,49; 140,19; 132,88 (ArC-C), 126,41; 124,59 (ArCH); nicht detektierbar (N*C*H₂); 35,68; 34,87 (*C*(CH₃)₃); 31,61; 31,06; 31,02 (C(*C*H₃)₃); HRMS (ESI) ber. für [M+H]⁺ C₆₀H₈₈N₂O₄P₂: 963,6292; gef.: 963,6293; ber. für [M+Na]⁺ C₆₀H₈₈N₂O₄P₂: 985,6112; gef.: 985,6117; Elementaranalyse ber. für C₆₀H₈₈N₂O₄P₂ (%): C 74,81 (75,06); H 9,21 (9,26); N 2,91 (2,72); P 6,42 (6,32).

### 1,4-Bis(dibenzo[d,f][1,3,2]dioxaphosphepin-6-yl)piperazin (5).

Ausbeute: 55%; weiße Kristalle; Schmp. 209-210 °C; R*_{f}*-Wert 0,35 (System *A₁*); ³¹P-NMR (CD₂Cl₂): δ 146,0; ¹H-NMR (CD₂Cl₂; 300,13 MHz): δ 7,49 (dd, 4H, *J*_{H,H} = 7,64 Hz; *J*_{H,H} = 1,74 Hz; ArH); 7,42-7,37; 7,31-7,19 (2m, 12H, ArH); 3,05-3,03 (m, 8H, NC*H*₂); ¹³C-NMR (CD₂Cl₂; 75,46 MHz): δ 151,56 (d, ²*J*_{C,P} = 4,37 Hz; Ar*C*-OP); 131,38 (d, ³*J*_{C,P} = 2,91 Hz; Ar*C*-C); 130,02; 129,65; 125,06; 122,21 (ArCH); 45,90-45,58 (m, ²*J*_{C,P}= 18,94 Hz; ³*J*_{C,P} = 4,37 Hz; N*C*H₂); HRMS (ESI) ber. für [M+H]⁺ C₂₈H₂₄N₂O₄P₂: 515,1284; gef.: 515,1283; Elementaranalyse ber. für C₂₈H₂₄N₂O₄P₂ (%): C 65,37 (65,51); H 4,70 (4,73); N 5,45 (5,52); P 12,04 (11,88).

### Tetrakis(2,4-di-tert-butylphenyl) piperazin-1,4-diylbis(phosphonit) (6).

Vgl. M. Fryberg, V. Weiss, EP 0070254 A1. (1983).

Ausbeute: 62,5%; weiße Kristalle; Schmp. 174-175 °C; R*_{f}*-Wert 0,2 (System *A₂*); ³¹P-NMR (CD₂Cl₂): δ 133,3; ¹H-NMR (CD₂Cl₂; 300,13 MHz): δ 7,39 (d, 4H, *J*_{H,H} = 2,36 Hz; ArH); 7,10 (qd, 8H, *J*_{H,H} = 8,40 Hz; *J*_{H,H} = 2,40 Hz; ArH); 3,37-3,36 (m, 8H, NC*H*₂); 1,43; 1,33 (2s, 72H, C(C*H*₃)₃); ¹³C-NMR(CD₂Cl₂; 100,61 MHz): δ 151,10 (d, ²*J*_{C,P}=8,37 Hz; Ar*C*-OP); 144,85; 131,33 (Ar*C*-C); 124,66; 123,73 (Ar*C*H); 117,79 (d, ³*J*_{C,P} = 22,31 Hz; Ar*C*-H); 45,11-44,86 (m, ²*J*_{C,P}= 19,52 Hz; ³*J*_{C,P} = 5,58 Hz; N*C*H₂); 35,33; 34,67 (*C*(CH₃)₃); 31,61; 30,23 (C(*C*H₃)₃); HRMS (ESI) ber. für [M+H]⁺ C₆₀H₉₂N₂O₄P₂: 967,6605; gef.: 967,6622; ber. für [M+Na]⁺ C₆₀H₉₂N₂O₄P₂: 989,6425; gef.: 989,6432; Elementaranalyse ber. für C₆₀H₉₂N₂O₄P₂(%): C 74,50 (74,53); H 9,59 (9,55); N 2,90 (2,91); P 6,40 (6,46). Einkristalle wurden durch langsames Verdampfen einer konzentrierten Lösung von Dichlormethan erhalten.

### Synthese von Phosphoramidit 7. Allgemeines Verfahren.

Vgl Y.H. Choi, J.Y. Choi, H.Y. Yang, Y.H. Kim, Tetrahedron: Asymmetry. 13 (2002) 801-804; M. Vuagnoux-d'Augustin, A. Alexakis, Chem. Eur. J. 13 (2007) 9647-9662; S. Lühr, J. Holz, A. Börner, ChemCatChem. 3 (2011) 1708-1730.

Eine Lösung des Amins (1,0 mmol) und Triethylamin (5,0 mmol) in THF (5 ml) wird bei 0 °C tropfenweise zu Phosphortrichlorid (2,0 mmol) zugegeben. Die Reaktionsmischung ließ man auf Raumtemperatur erwärmen und weitere drei Stunden rühren. Das resultierende HCl-Gas wurde aus dem Reaktionsgefäß unter Verwendung eines leichten Argonstroms ausgetrieben. Die klare Lösung wird eingeengt und azeotrop mit Toluol (dreimal) getrocknet. Der erhaltene Rückstand wurde direkt im nächsten Schritt ohne weitere Reinigung verwendet. Das ölige Rohprodukt wurde in THF (20 ml) gelöst und auf 0 °C gekühlt. Eine Lösung von Phenol (4,0 mmol) und Triethylamin (5,0 mmol) in THF (5 ml) wurde dann tropfenweise zu der gerührten Lösung zugegeben. Die Reaktionsmischung wurde langsam auf Raumtemperatur gebracht und das Rühren über Nacht fortgesetzt. Der Niederschlag wurde abfiltriert und die erhaltene Lösung eingeengt. Die Rohprodukte wurden durch Flash-Chromatrography gereinigt und das reine Amidite **7** als weißer Feststoff erhalten.

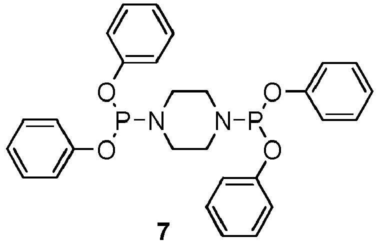

### Tetraphenyl piperazin-1,4-diylbis(phosphonit) (7).

Vgl. Zh. Beishekeev, B. Ashimbaeva, T. Chyntemirova, K. Dzhundubaev, Zh. Anyrova, T. Toktobekova, Izvestiya Akademii Nauk Kirgizskoi SSR. 1 (1980) 37-39.

Ausbeute: 61%; weiße Kristalle; Schmp. 110-111 °C; R*_{f}*-Wert 0,40 (System *A₁*); ³¹P-NMR (CD₂Cl₂): δ 136,94; ¹H-NMR (CD₂Cl₂; 300,13 MHz): δ 7,35-7,28; 7,12-7,04 (2m, 20H, ArH); 3,25-3,22 (m, 8H, NC*H*₂); ¹³C-NMR (CD₂Cl₂; 75,46 MHz): δ 154,01 (d, ²*J*_{C,P} = 6,25 Hz; Ar*C*-OP); 130,02; 123,55; 120,54; 120,41 (Ar*C*H, 2 Signale sind isochron); 44,94-44,63 (m, ²*J*_{C,P} = 18,21 Hz; ³*J*_{C,P} = 4,79 Hz; N*C*H₂); HRMS (ESI) ber. für [M+H]⁺ C₂₈H₂₈N₂O₄P₂: 519,15971; gef.: 519,16035; ber. für [M+Na]⁺ C₂₈H₂₈N₂O₄P₂: 541,14165; gef.: 541,1416; Elementaranalyse ber. für C₂₈H₂₈N₂O₄P₂ (%): C 64,86 (64,89); H 5,44 (5,45); N 5,40 (5,59); P 11,95 (11,83).

### Synthese Rh (I) -Komplexe 8 und 9.

Zu einer Lösung von Rh(acac)(CO)₂ (0,2 mmol) in Toluol (5 ml) tropf man bei Raumtemperatur unter Rühren eine Lösung des Liganden (0,1 mmol) in Toluol (5 ml) innerhalb von 10 min. Nach vollendeter Zugabe wird zwei Stunden gerührt und die Reaktionslösung im Vakuum eingeengt. Durch waschen des Rückstandes mit Hexan (6 ml) und trocknen über drei Stunde bei 60 °C, ergeben die spektroskopisch reinen Produkte.

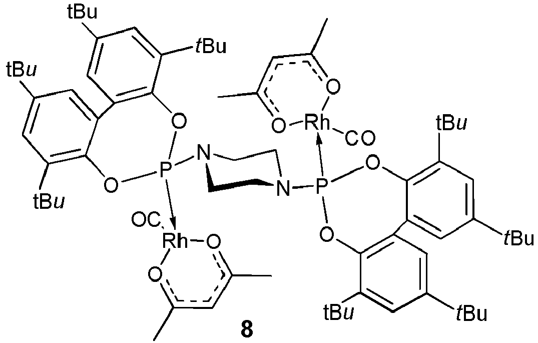

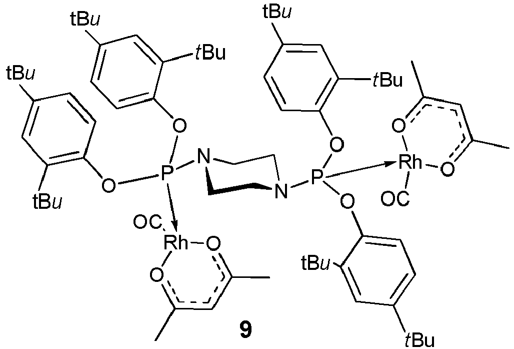

**Abbildung 5.** Rh-Komplexe **8** und **9.**

### [Rh(acac)(CO)]₂(4)Komplex (8).

Ausbeute: quantitativ; gelbes Pulver; ³¹P-NMR (CD₂Cl₂): δ 141,80 (br. d, ¹*J*_{P,Rh} = 276,95 Hz); IR: ν (CO) 2000,3 cm⁻¹; ¹H-NMR (THF-d8; 300,13 MHz): δ 7,37 (d, 4H, *J*_{H,H} = 1,83 Hz; ArH); 7,04 (br. s, 4H, *J*_{H,H} = 1,64 Hz; ArH); 5,34 (s, 2H, C*H*_{acac}); 3,03-2,28 (m, 8H, NC*H*₂); 1,83 (s, 6H, Me_{acac}); 1,77 (br. s, 6H, Me_{acac}); 1,41; 1,23 (2s, 72H, C(C*H*₃)₃); ¹³C-NMR (THF-d8; 75,46 MHz): δ 188,56; 187,58 (2d, *J*_{C,Rh} = 29,15 Hz; *C*O); 187,34; 183,73 (*C*O_{acac}); 146,28 (Ar*C*-OP); 145,87 (d, *J*_{C,P} = 9,48 Hz; Ar*C*-C); 149,51; 131,28; 127,16; 124,22 (ArCH); 99,54 (*C*H_{acac}); nicht detektierbar (N*C*H₂); 34,95; 33,89 (*C*(CH₃)₃); 30,62; 30,39 (C(*C*H₃)₃); 26,51 (Meacac); 26,02 (d, ⁵*J*_{C,Rh} = 8,16 Hz; Me_{acac}); HRMS (ESI) ber. für [M+Na-2H]⁺ C₇₂H₁₀₂N₂O₁₀P₂Rh₂: 1445,2015; gef.: 1445,50111; Elementaranalyse ber. für C₇₂H₁₀₄N₂O₁₀P₂Rh₂ (%): C 60,67 (60,82); H 7,35 (7,33); N 1,97 (1,93); P 4,35 (4,45); Rh 14,44 (14,30).

### [Rh(acac)(CO)]₂(6)Komplex (9).

Ausbeute: quantitativ; gelbes Pulver; ³¹P-NMR (CD₂Cl₂): δ 128,70 (d, ¹*J*_{P,Rh} = 258,56 Hz); IR: ν (CO) 1992,2 cm⁻¹; ¹H-NMR (CD₂Cl₂; 300,13 MHz): δ 7,49 (dd, 4H, *J*_{H,H} = 8,45 Hz; *J*_{H,H} = 1,41 Hz; ArH); 7,31 (br. d, 4H, *J*_{H,H} = 1,64 Hz; ArH); 7,04 (dd, 4H, *J*_{H,H} = 8,45 Hz; *J*_{H,H} = 2,46 Hz; ArH); 5,35 (s, 2H, C*H*_{acac}); 3,70-3,04 (m, 8H, NC*H*₂); 1,89; 1,52 (2s, 12H, Me_{acac}); 1,29; 1,23 (2s, 72H, C(C*H*₃)₃); ¹³C-NMR (CD₂Cl₂; 75,46 MHz): δ 188,56; 187,58 (2d, *J*_{C,Rh} = 31,58 Hz; *C*O); 188,29; 185,84 (*C*O_{acac}); 149,53 (d, ²*J*_{C,P}=3,20 Hz; Ar*C*-OP); 146,01 (Ar*C*-C); 138,89 (d, ³*J*_{C,P} = 5,88 Hz; Ar*C*-C); 124,77; 123,35(Ar*C*H); 119,75 (*J*_{C,P} = 9,61 Hz; ArCH); 101,03 (*C*H_{acac}); 46,86 (N*C*H₂); 35,31; 34,77 (*C*(CH₃)₃); 31,68; 30,32 (C(*C*H₃)₃); 27,60 (d, ⁵*J*_{C,Rh} = 7,68 Hz; Me_{acac}); 26,84 (Me_{acac}); HRMS (ESI) ber. für [M+Na-2H]⁺ C₇₂H₁₀₆N₂O₄P₂Rh₂: 1449,5325; gef.: 1449,5297; Elementaranalyse ber. für C₇₂H₁₀₈N₂O₄P₂Rh₂ (%): C 60,50 (60,62); H 7,62 (7,52); N 1,96 (1,91); P 4,33 (4,40); Rh 14,40 (14,36).

### Hydroformylierungsverfahren

Die Hydroformylierungsversuche wurden in einem 200 ml-Autoklaven, der mit einem Thermoelement, einem Bronkhorst HITEC Massendurchflussmesser und ein Bronkhorst Druckregler ausgestattet ist, bei 120 °C und einem Druck von 50 bar Synthesegas (99,997%; CO/H₂ = 1:1) durchgeführt. Die Reaktion erfolgte bei konstantem Druck über einen Zeitraum von vier Stunden. Der Autoklav mit dem Speicherbehälter für den Olefin-Zusatz wurde mit Argon mehrmals gespült, bevor die Katalysatorlösung (= Metallkomplex + Ligand + Lösungsmittel) in den Reaktor und das Olefin in den Vorratsbehälter eingeführt wurde (Argon Gegenstrom). In einem typischen Experiment wurden Olefin (15 ml) und Katalysatorlösung (41 ml) mit einem Olefin/Rhodium-Verhältnis von 2000/1 verwendet. Die Katalysatorlösung wurde unter Synthesegas auf die gewünschte Reaktionstemperatur für 30 Minuten erhitzt. Nach der Olefinzugabe wurde der Druck bei 50 bar gehalten und der Gasverbrauch wurde mit einem Massendurchflussmesser gemessen. Nach vier Stunden wurde der Autoklav auf Raumtemperatur abgekühlt und der Druck abgelassen. Die Produktanalyse erfolgte durch Gaschromatographie, zu diesem Zweck wurde die Reaktionslösung (1 ml) mit n-Pentan (10 ml) verdünnt und Toluol als interner Standard verwendet.

Versuche wurden jeweils mit n-Octenen (EVONIK Industries AG, Octenisomerengemisch aus 1-Octen: 3,3 %; cis+trans-2-Octen: 48.5%; cis+trans-3-Octen: 29.2%; cis+trans-Octen-4: 16.4%; gerüstisomere Octene: 2.6%), 1-Octen bzw. 2-Penten als Edukten durchgeführt. Die dabei erzielten Ausbeuten und n/iso-Verhältnisse sind in den Tabellen 1 bis 3 dargestellt. Wie aus den Versuchsergebnissen hervorgeht, eignen sich die erfindungsgemäßen Komplexe als Katalysatoren für die Hydroformylierung von Olefinen, mit denen sich nahezu quantitative Ausbeuten erzielen lassen. Zudem lässt sich durch Auswahl der erfindungsgemäßen Liganden eine hohe n- bzw. iso-Selektivität erzielen.

**Tabelle 1**

| Hydroformylierung von n-Octen mit den zweizähnigen Diphosphoramiditen **4** und **6.** | | | | |
|---|---|---|---|---|
| Eintrag | Ligand | L/Rh | Ausbeute (%) | *n*-Selektivität (%) |
| 1 | 4 | 2 | 98,9 | 18,7 |
| 2 | 6 | 2 | 91,7 | 16,9 |

**Tabelle 2**

| Hydroformylierung von 1-Octen mit den zweizähnigen Diphosphoramiditen **4, 5** und **7.** | | | | |
|---|---|---|---|---|
| Eintrag | Ligand | L/Rh | Ausbeute (%) | *n*-Selektivität (%) |
| 1 | 4 | 2 | 99,6 | 52,8 |
| 2 | 5 | 2 | 85,3 | 75,2 |
| 3 | 7 | 2 | 28,5 | 69,0 |

**Tabelle 3**

| Hydroformylierung von 2-Penten mit den zweizähnigen Diphosphoramiditen **5** und **6.** | | | | |
|---|---|---|---|---|
| Eintrag | Ligand | L/Rh | Ausbeute (%) | *n*-Selektivität (%) |
| 1 | 5 | 2 | 59,4 | 20,3 |
| 2 | 6 | 2 | 100 | 36,6 |

Wie die Ergebnisse zeigen kann mit Hilfe der neun Liganden das n/iso-Verhältnis gesteuert werden.

## Patentansprüche

1. Komplex umfassend Rh, Ru, Co oder Ir und eine Verbindung nach einer der allgemeinen Formeln (**I**) oder (**II**) 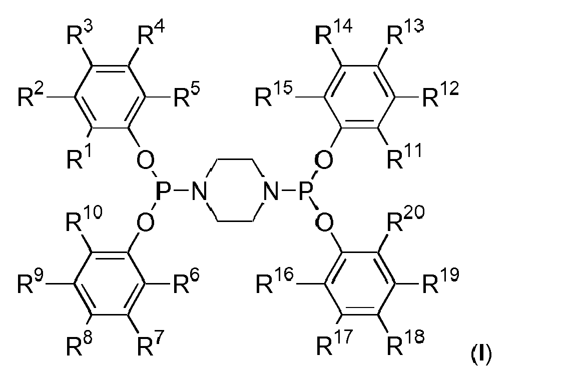 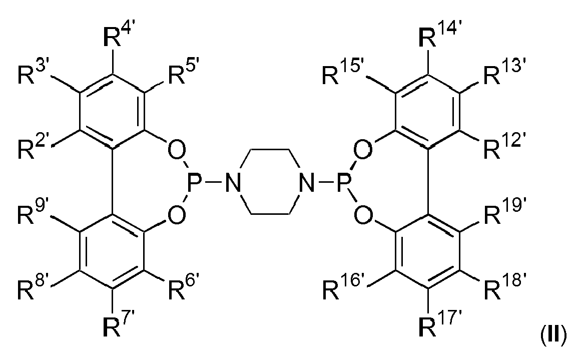 wobei R¹, R², R³, R⁴, R⁵, R⁹, R⁷, R⁸, R⁹, R¹⁰, R¹¹, R¹², R¹³, R¹⁴, R¹⁵, R¹⁶, R¹⁷, R¹⁸, R¹⁹, R²⁰ unabhängig voneinander ausgewählt sind aus -H, -(C₁-C₁₂)-Alkyl, -O-(C₁-C₁₂)-Alkyl, -(C₆-C₂₀)-Aryl, -O-(C₆-C₂₀)-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -N[(C₁-C₁₂)-Alkyl]₂; und R^{2'}, R^{3'}, R^{4'}, R^{5'}, R^{6'}, R^{7'}, R^{8'}, R^{9'}, R^{12'}, R^{13'}, R^{14'}, R^{15'}, R^{16'}, R^{17'}, R^{18'}, R^{19'} unabhängig voneinander ausgewählt sind aus -H, -(C₁-C₁₂)-Alkyl, -O-(C₁-C₁₂)-Alkyl,-(C₆-C₂₀)-Aryl, -O-(C₆-C₂₀)-Aryl, Halogen, -COO-(C₁-C₁₂)-Alkyl, -CONH-(C₁-C₁₂)-Alkyl,-CO-(C₁-C₁₂)-Alkyl, -CO-(C₆-C₂₀)-Aryl, -COOH, -OH, -N[(C₁-C₁₂)-Alkyl]₂.

2. Komplex nach Anspruch 1,
umfassend Rh.

3. Komplex nach einem der Ansprüche 1 und 2,
**dadurch gekennzeichnet, dass** R¹, R², R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰ unabhängig voneinander ausgewählt sind aus -H, -(C₁-C₁₂)-Alkyl, -O-(C₁-C₁₂)-Alkyl, -(C₆-C₂₀)-Aryl, -O-(C₆-C₂₀)-Aryl, - Halogen.

4. Komplex nach einem der Ansprüche 1 bis 3,
**dadurch gekennzeichnet, dass** R¹, R², R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰ unabhängig voneinander ausgewählt sind aus -H, -(C₁-C₁₂)-Alkyl, -O-(C₁-C₁₂)-Alkyl, -(C₆-C₂₀)-Aryl, -O-(C₆-C₂₀)-Aryl.

5. Komplex nach einem der Ansprüche 1 bis 4,
**dadurch gekennzeichnet, dass** R^{2'}, R^{3'}, R^{4'}, R^{5'}, R^{6'}, R^{7'}, R^{8'}, R^{9'}, R^{12'}, R^{13'}, R^{14'}, R^{15'}, R^{16'}, R^{17'}, R^{18'}, R^{19'} unabhängig voneinander ausgewählt sind aus -H, -(C₁-C₁₂)-Alkyl,-O-(C₁-C₁₂)-Alkyl, -(C₆-C₂₀)-Aryl, -O-(C₆-C₂₀)-Aryl, - Halogen.

6. Komplex nach einem der Ansprüche 1 bis 5,
**dadurch gekennzeichnet, dass** R^{2'}, R^{3'}, R^{4'}, R^{5'}, R^{6'}, R^{7'}, R^{8'}, R^{9'}, R^{12'}, R^{13'}, R^{14'}, R^{15'}, R^{16'}, R^{17'}, R^{18'}, R^{19'} unabhängig voneinander ausgewählt sind aus -H, -(C₁-C₁₂)-Alkyl, - O-(C₁-C₁₂)-Alkyl, -(C₆-C₂₀)-Aryl, -O-(C₆-C₂₀)-Aryl.

7. Komplex nach einem der Ansprüche 1 bis 6,
**dadurch gekennzeichnet, dass** R¹, R², R⁴, R⁷, R⁹, R¹⁰, R¹¹, R¹², R¹⁴, R¹⁷, R¹⁹, R²⁰ jeweils für H stehen.

8. Komplex nach einem der Ansprüche 1 bis 7,
**dadurch gekennzeichnet, dass** R^{2'}, R^{4'}, R^{7'}, R^{9'}, R^{12'}, R^{14'}, R^{17'}, R^{19'} jeweils für H stehen.

9. Komplex nach einem der Ansprüche 1 bis 8,
**dadurch gekennzeichnet, dass** die Verbindung nach einer der Formeln (**I**) und (**II**) ausgewählt ist aus den Verbindungen (**4**), (**5**), (**6**) und (**7**)

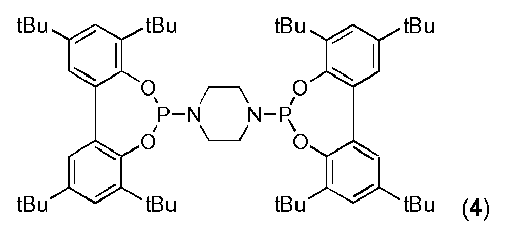

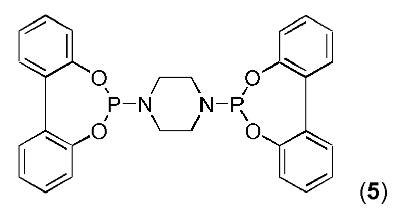

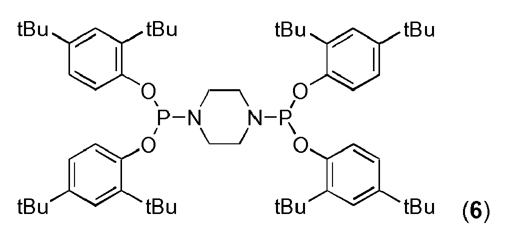

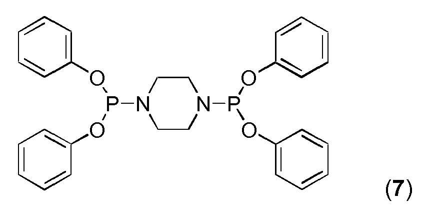

10. Verwendung eines Komplexes nach einem der Ansprüche 1 bis 9 zur Katalyse einer Hydroformylierungsreaktion.

11. Verfahren zur Herstellung eines Aldehyds, umfassend die Verfahrensschritte:
a) Vorlegen eines Olefins,
b) Zugabe eines Komplexes nach einem der Ansprüche 1 bis 9 oder einer Verbindung nach einer der Formeln (**I**) oder (**II**) gemäß einem der Ansprüche 1 bis 9 und einer Katalysatorvorstufe umfassend einen Rh-, Ru-, Co- oder Ir-Komplex,
c) Zuführen von Wasserstoff und Kohlenstoffmonoxid,
d) Erwärmen des Reaktionsgemisches, wobei das Olefin zu einem Aldehyd umgesetzt wird.

12. Verfahren nach Anspruch 11,
**dadurch gekennzeichnet, dass** die Katalysatorvorstufe einen Liganden ausgewählt aus Acetylacetonat, Acetat und Chlorid umfasst.

13. Verfahren nach einem der Ansprüche 11 und 12,
**dadurch gekennzeichnet, dass** die Katalysatorvorstufe Rh(acac)(CO)₂ ist.
